# FASCICULE DE BREVET EUROPEEN

(11) **EP 0 918 741 B1**
(45) Date de publication et mention de la délivrance du brevet: **09.05.2001**
(21) Numéro de dépôt: 97926056.9
(22) Date de dépôt: 29.05.1997
(51) Int. Cl.: C07C 55/14, C07C 51/43

(54) **PROCEDE DE PURIFICATION DE L'ACIDE ADIPIQUE PAR CRISTALLISATION**
VERFAHREN ZUR REINIGUNG DER ADIPIQSÄURE DURCH KRISTALLISATION
METHOD FOR PURIFYING ADIPIC ACID BY CRYSTALLIZATION

(30) Priorité: 04.06.1996 FR 9607171
(43) Date de publication de la demande: 02.06.1999
(73) Titulaire: RHODIA POLYAMIDE INTERMEDIATES, 69190 Saint-Fons (FR)
(72) Inventeur: HENRIET, Eric, B., F-69003 Lyon (FR); LECONTE, Philippe, F-69330 Meyzieu (FR); PATOIS, Carl, 68400 Riedisheim (FR); PERRON, Robert, F-69390 Charly (FR)
(74) Mandataire: Esson, Jean-Pierre
(86) Numéro de dépôt international: FR9700939
(87) Numéro de publication internationale: WO9746510

(56) Documents cités:
- FR-A- 901 841
- FR-A- 1 349 134
- US-A- 2 862 027

## Description

La présente invention concerne un procédé de purification de l'acide adipique par cristallisation ou recristallisation dans au moins un acide carboxylique.

L'acide adipique est l'une des deux matières de base de la préparation du polyamide 6-6. Pour les applications du polyamide 6-6, il est nécessaire d'avoir une très grande pureté et cette pureté doit déjà exister au stade des précurseurs, notamment au stade de l'acide adipique.

Selon le procédé de préparation de l'acide adipique, les impuretés qu'il recèle sont évidemment différentes. Le présent procédé peut s'appliquer à l'acide adipique provenant des divers procédés de synthèse. En effet, une des impuretés les plus gênantes et parfois les plus coûteuses est constituée par la présence de traces du catalyseur utilisé lors de la préparation de l'acide adipique.

Cependant dans l'exposé qui suit, le procédé sera appliqué plus particulièrement à l'acide adipique issu de la double hydroxycarbonylation du butadiène ou encore issu de l'oxydation du cyclohexane.

La première hydroxycarbonylation du butadiène conduit à un mélanges d'acides penténoïques, principalement à l'acide 3-penténoïque. La deuxième hydroxycarbonylation porte sur les acides penténoïques obtenus dans la première réaction et conduit à l'acide adipique contenant également une certaine quantité d'acide méthyl-2 glutarique, d'acide éthyl-2 succinique, ainsi que d'autres composés, provenant déjà de la première réaction d'hydroxycarbonylation, tels que la gamma-valérolactone, les acides penténoïques non transformés, l'acide méthylbuténoïque. Il contient aussi des traces du catalyseur utilisé dans la deuxième réaction d'hydroxycarbonylation, le plus souvent l'iridium et/ou le rhodium.

L'oxydation directe du cyclohexane en acide adipique est généralement réalisée en présence de cobalt et dans ce procédé l'acide adipique obtenu contient des traces de catalyseur au cobalt.

L'acide adipique étant peu soluble à froid et beaucoup plus soluble à chaud dans l'eau, ce solvant est généralement utilisé pour la cristallisation dudit acide.

Cependant, compte-tenu des puretés très grandes exigées de plus en plus pour l'acide adipique, notamment en ce qui concerne les traces métalliques, une ou même plusieurs recristallisations dans l'eau s'avèrent souvent insuffisantes.

Outre la gêne que peut occasionner la présence de traces métalliques pour les diverses utilisations de l'acide adipique, la valeur elle-même de certains catalyseurs comme l'iridium ou le rhodium, compte-tenu des tonnages très importants de l'acide adipique, fait que leur récupération la plus poussée possible est indispensable dans le cadre d'un procédé industriel économiquement viable.

La présente invention consiste en un procédé amélioré de cristallisation ou de recristallisation de l'acide adipique, caractérisé en ce que ladite cristallisation ou recristallisation est effectuée dans au moins un acide carboxylique ayant un point de fusion inférieur à 20 °C, et en ce que l'acide adipique soumis à la cristallisation a une purté minimale de 95 %.

Les acides carboxyliques mis en oeuvre dans le présent procédé sont plus particulièrement les acides carboxyliques aliphatiques, saturés ou comportant une insaturation éthylénique.

Ce sont de préférence les acides monocarboxyliques, linéaires ou ramifiés, ayant de 2 à 6 atomes de carbone.

A titre d'exemples non limitatifs de tels acides monocarboxyliques, on peut citer l'acide acétique, l'acide propionique, les acides butanoïques, les acides pentanoïques, les acides hexanoïques, les acides penténoïques.

L'acide acétique et les acides penténoïques sont préférés, l'acide acétique en raison de sa disponibilité et de son utilisation dans la synthèse de l'acide adipique à partir du cyclohexane, et les acides penténoïques parce qu'ils sont un intermédiaire dans la préparation de l'acide adipique à partir du butadiène.

La pureté de l'acide adipique ainsi recristallisé peut encore être améliorée lorsque la recristallisation est effectuée en présence de monoxyde de carbone.

Le monoxyde de carbone peut constituer au moins en partie l'atmosphère surmontant la solution dans le réacteur de cristallisation ou de recristallisation (ou ciel du réacteur) ou créer dans ledit réacteur une pression supérieure à la pression atmosphérique.

En pratique, on opérera donc sous une pression absolue de 0 bar (de préférence au moins 0,5 bar) à 50 bars de monoxyde de carbone, la limite supérieure n'ayant pas de caractère critique, mais étant représentative d'un appareillage industriel qui ne soit pas excessivement coûteux.

L'acide adipique brut soumis à la recristallisation selon le présent procédé est habituellement un acide adipique ayant déjà subi un ou plusieurs traitements de purification, notamment par cristallisation dans l'eau, par raffinage ou encore par distillation, afin d'avoir une pureté minimale de 95 % .

Généralement l'acide adipique que l'on recristallise par le procédé de l'invention a une pureté de 95 % à 99,95 %.

La recristallisation consiste à dissoudre l'acide adipique à purifier dans le minimum d'acide carboxylique aliphatique à chaud, c'est-à-dire habituellement à une température de 80°C à 250°C, éventuellement sous atmosphère ou pression au moins partielle de monoxyde de carbone, puis à faire cristalliser l'acide adipique dissous par un refroidissement de la solution, éventuellement après un ensemencement de la solution à l'aide de cristaux d'acide adipique pur.

Généralement la quantité d'acide carboxylique utilisée est celle qui conduit à une solution saturée d'acide adipique à la température choisie. A titre indicatif, à 90°C la solution saturée dans l'acide 3-penténoïque comporte environ 33 % d'acide adipique en poids par poids.

La teneur en catalyseur de l'acide adipique peut également être diminuée lorsque l'on réalise la recristallisation en présence d'un acide protonique fort.

Par acide protonique fort, on entend dans le présent texte un acide protonique minéral ayant un pKa inférieur à 1.

A titre d'exemples non limitatifs de tels acides protoniques forts, on peut citer l'acide iodhydrique, l'acide bromhydrique, l'acide chlorhydrique, l'acide nitrique, l'acide sulfurique.

La quantité d'acide protonique fort peut varier de 0 mole à 100 moles par mole de métal catalyseur compris dans l'acide adipique. De préférence, la quantité d'acide protonique varie de 0 mole (encore plus préférentiellement de 1 mole) à 50 moles par mole de métal catalyseur.

Le procédé de l'invention comprend également la cristallisation de l'acide adipique à partir des mélanges réactionnels qui le contiennent.

C'est ainsi que l'on peut par exemple cristalliser l'acide adipique à partir du mélange obtenu par hydroxycarbonylation d'acide penténoïque par l'eau et le monoxyde de carbone. Ce mélange réactionnel peut être mélangé avec l'acide carboxylique, en présence ou non du monoxyde de carbone utilisé pour la réaction d'hydroxycarbonylation, et l'ensemble être maintenu à une température de 80°C à 250°C, comme indiqué précédemment pour la recristallisation.

On peut également effectuer la cristallisation dans le réacteur d'hydroxycarbonylation, en laissant refroidir le mélange réactionnel, de préférence sous pression de monoxyde de carbone. Cette variante peut être mise en oeuvre notamment lorsque l'hydroxycarbonylation est conduite dans un acide carboxylique ou lorsqu'elle est conduite dans l'acide 3-penténoïque avec un taux de transformation de ce dernier qui ne soit pas complet.

La réaction d'hydroxycarbonylation étant conduite en présence de monoxyde de carbone, il n'est généralement pas nécessaire de rajouter ce composé pour la cristallisation, mais le cas échéant cette possibilité n'est pas exclue.

De même, le promoteur utilisé dans la réaction d'hydroxycarbonylation pouvant être l'acide iodhydrique ou l'acide bromhydrique, il peut ne pas être nécessaire de rajouter de l'acide protonique fort. Cependant, on peut si on le souhaite compléter la quantité d'acide protonique fort présente dans le mélange réactionnel. On peut également comme pour la recristallisation de l'acide adipique opérer en l'absence d'acide protonique fort, bien que cette variante ne soit pas préférée.

La recristallisation selon l'invention peut être effectuée plusieurs fois successivement sur l'acide adipique, afin de diminuer encore la teneur en métal catalyseur. On peut également faire suivre une cristallisation ou recristallisation selon l'invention par une ou plusieurs recristallisations dans l'eau.

Les exemples qui suivent illustrent l'invention.

### EXEMPLE 1

Dans une ampoule de verre, on charge 5,2 g d'acide adipique contenant 31,2 µg de Co (0,0006 % en poids par poids d'acide adipique) et 7,5 ml d'acide acétique. L'acide adipique a été préparé par oxydation directe du cyclohexane en présence d'acétate de Co et a été purifié par recristallisation dans l'eau. Il ne contient pas d'impuretés organiques en quantités dosables.

L'ampoule ouverte est placée dans un autoclave de 125 ml que l'on ferme.

On met à froid un ciel d'azote (1 bar environ).

On chauffe à 185°C et on maintient environ 30 min à cette température.

Après refroidissement et purge de l'autoclave à l'azote, on filtre l'acide adipique et on rince l'autoclave avec quelques ml d'acide acétique.

On lave l'acide adipique filtré avec 2 fois 5 ml d'acide acétique, puis avec 3 fois 8 ml d'acide acétique.

On sèche l'acide adipique pendant une nuit à l'étuve (60°C). On dose le cobalt présent dans l'acide adipique final par plasma à induction couplée, couplé avec la spectrométrie de masse (IPC/Masse). On trouve 0,00008 % de Co en poids par pcids.

### EXEMPLE 2

On répète l'exemple 1 avec les mêmes charges et dans les mêmes conditions opératoires, mais en ajoutant au mélange réactionnel mis en oeuvre 10 équivalents molaires de HCI par équivalent molaire de Co présent dans l'acide adipique engagé.

Après le même traitement que pour l'exemple 1, on obtient un acide adipique final sec comprenant 0,000009 % de Co en poids par poids.

### EXEMPLES 3 A 8

On répète l'exemple 1 dans les mêmes conditions opératoires, mais en ajoutant au mélange réactionnel mis en oeuvre HI (avec un rapport molaire HI/Ir indiqué dans le tableau 1 ci-après), en utilisant l'acide 3-penténoïque (P3) comme solvant de recristallisation et en mettant en oeuvre un acide adipique (AdOH) comportant de l'iridium. L'acide adipique a été préparé par hydroxycarbonylation de l'acide 3-penténoïque en présence d'un catalyseur à base d'lr et a été purifié par recristallisation dans l'eau. Il ne contient pas d'impuretés organiques en quantités dosables.

Après la recristallisation, l'acide adipique est lavé avec de l'acide 3-penténoïque saturé en acide adipique, puis par de l'eau saturée en acide adipique.

Le tableau 1 ci-après rassemble les conditions dans lesquelles ont été réalisés les exemples (Tp = température) ainsi que les teneurs initiales et finales en Ir (Ir initial et Ir final) exprimées en microgrammes par gramme, de l'acide adipique mis en oeuvre.

**Tableau 1**

| **Exemple** | **AdOH en g** | **P3 en g** | **Tp en °C** | **Durée en min** | **CO en bars** | **Rapport molaire Hl/lr** | **Ir initial** | **Ir final** |
|---|---|---|---|---|---|---|---|---|
| Ex 3 | 2,7 | 3,9 | 185 | 20 | 30 | 10 | 5,0 | 0,76 |
| Ex 4 | 1,43 | 2,0 | 90 | 120 | 30 | 10 | 5,0 | 2,6 |
| Ex 5 | 5,2 | 7,8 | 185 | 30 | 30 | 20 | 2,2 | 0,42 |
| Ex 6 | 6,2 | 9,3 | 185 | 30 | 1 | 20 | 2,2 | 0,56 |
| Ex 7 | 2,9 | 4,5 | 185 | 1200 | 1 | 10 | 0,46 | 0,18 |
| Ex 8 | 8,05 | 13 | 185 | 40 | 1 | 20 | 8,0 | 1,3 |

### EXEMPLES 9 A 12

On répète les exemples 3 à 8, mais en mettant en oeuvre un acide adipique (AdOH) comportant du rhodium. L'acide adipique a été préparé par hydroxycarbonylation de l'acide 3-penténoïque en présence d'un catalyseur à base de Rh et a été purifié par recristallisation dans l'eau. Il ne contient pas d'impuretés organiques en quantités dosables.

Après la recristallisation, l'acide adipique est lavé avec de l'acide 3-penténoïque saturé en acide adipique, puis par de l'eau saturée en acide adipique.

Le tableau 2 ci-après rassemble les conditions dans lesquelles ont été réalisés les exemples (Tp = température) ainsi que les teneurs initiales et finales en Rh (Rh initial et Rh final) exprimées en microgrammes par gramme, de l'acide adipique mis en oeuvre.

**Tableau 2**

| **Exemple** | **AdOH en g** | **P3 en g** | **Tp en °C** | **Durée en min** | **CO en bars** | **Rapport molaire HI/Rh** | **Rh initial** | **Rh final** |
|---|---|---|---|---|---|---|---|---|
| Ex 9 | 4,99 | 7,5 | 190 | 30 | 30 | 10 | 9,5 | 3,2 |
| Ex 10 | 5,02 | 7,5 | 190 | 30 | 1 | 13 | 9,5 | 3,1 |
| Ex 11 | 5,04 | 7,5 | 190 | 30 | 0 (1 bar argon) | 12 | 9,5 | 3,8 |
| Ex 12 | 5,05 | 7,5 | 190 | 600 | 1 | 14 | 9,5 | 0,46 |

### EXEMPLE 13

Dans l'exemple 13, on répète la recristallisation décrite pour les exemples 3 à 8, en mettant en oeuvre l'acide adipique recristallisé obtenu dans l'exemple 7.

Le tableau 3 ci-après rassemble les conditions dans lesquelles a été réalisé l'exemple ainsi que les teneurs initiales et finales en Ir (Ir initial et Ir final) exprimées en microgrammes par gramme, de l'acide adipique mis en oeuvre.

**Tableau 3**

| **Exemple** | **AdOH en g** | **P3 en g** | **Tp en °C** | **Durée en min** | **CO en bars** | **Rapport molaire HI/Ir** | **Ir initial** | **Ir final** |
|---|---|---|---|---|---|---|---|---|
| Ex 13 | 1,26 | 2,0 | 185 | 30 | 1 | 30 | 0,18 | 0,14 |

### ESSAI COMPARATIF 1

On effectue une recristallisation dans l'eau d'un acide adipique obtenu par hydroxycarbonylation de l'acide 3-penténoïque en présence d'iridium et de HI. Cet acide adipique a déjà subi une cristallisation et il contient encore 0,00022 % d'iridium.

La recristallisation s'effectue de manière classique par dissolution à 95°C environ de l'acide adipique dans le minimum d'eau, puis par refroidissement progressif de la solution obtenue, puis filtration, enfin lavage de l'acide adipique filtré avec 2 fois 5 ml d'eau et avec 3 fois 8 ml d'eau.

On sèche l'acide adipique pendant une nuit à l'étuve (60°C). On dose l'iridium présent dans l'acide adipique final. On trouve 0,00022 % de Ir en poids par poids). On n'a donc pas réussi à diminuer la teneur en iridium de l'acide adipique.

### EXEMPLE 14

On réalise l'hydroxycarbonylation de l'acide 3-penténoïque avec les charges suivantes :
- 77,7 g d'acide 3-penténoïque
- 88,1 mg de [IrCl(COD)]₂
- 169,6 mg d'une solution aqueuse à 57 % en poids de Hl
- 5,7 g d'eau en injection continue

La pression de monoxyde de carbone à la température de réaction (185°C) est maintenue à 22 bars et la réaction est arrêtée après une durée de 1 h 05 min.

Après refroidissement progressif sous la pression de monoxyde de carbone de 22 bars, on effectue le soutirage et la filtration de l'acide adipique cristallisé. Puis, par dosage par chromatographie en phase gazeuse et par chromatographie liquide à haute performance des composés du filtrat, on détermine un taux de transformation de l'acide 3-penténoïque de 56,6 %.

L'acide adipique ainsi cristallisé contient 0,000122 % d'iridium.

Cet acide adipique est recristallisé dans l'acide 3-penténoïque sous atmosphère de monoxyde de carbone (circulation au bulle à bulle).

Après la recristallisation, l'acide adipique est lavé avec de l'acide 3-penténoïque saturé en acide adipique, puis par de l'eau saturée en acide adipique.

L'acide adipique recristallisé et séché présente une teneur en iridium de 0,00002 % en poids par poids.

## Revendications

1. Procédé de cristallisation ou de recristallisation de l'acide adipique permettant de diminuer la teneur en traces métalliques, caractérisé en ce que ladite cristallisation ou recristallisation est effectuée dans au moins un acide carboxylique ayant un point de fusion inférieur à 20 °C et en ce que l'acide adipique soumis à la recristallisation a une pureté minimale de 95 % .

2. Procédé selon la revendication 1, caractérisé en ce que l'acide carboxylique mis en oeuvre est choisi parmi les acides carboxyliques aliphatiques, saturés ou comportant une insaturation éthylénique et de préférence parmi les acides monocarboxyliques, linéaires ou ramifiés, ayant de 2 à 6 atomes de carbone.

3. Procédé selon l'une des revendications 1 ou 2, caractérisé en ce que l'acide carboxylique mis en oeuvre est choisi parmi l'acide acétique, l'acide propionique, les acides butanoïques, les acides pentanoïques, les acides hexanoïques, les acides penténoïques.

4. Procédé selon l'une des revendications 1 à 3, caractérisé en ce que la cristallisation ou recristallisation est effectuée en présence de monoxyde de carbone.

5. Procédé selon la revendication 4, caractérisé en ce que le monoxyde de carbone constitue au moins en partie l'atmosphère surmontant la solution dans le réacteur de cristallisation ou de recristallisation ou crée dans ledit réacteur une pression supérieure à la pression atmosphérique.

6. Procédé selon l'une des revendications 4 ou 5, caractérisé en ce que l'on opère sous une pression absolue de 0,5 bar à 50 bars de monoxyde de carbone.

7. Procédé selon l'une des revendications 1 à 6, caractérisé en ce que l'acide adipique brut soumis à la recristallisation a une pureté de 95 % à 99,95 %.

8. Procédé selon l'une des revendications 1 à 7, caractérisé en ce que l'on réalise la recristallisation en présence d'un acide protonique fort.

9. Procédé selon la revendication 8, caractérisé en ce que l'acide protonique fort est choisi parmi l'acide iodhydrique, l'acide bromhydrique, l'acide chlorhydrique, l'acide nitrique, l'acide sulfurique.

10. Procédé selon l'une des revendications 1 à 9, caractérisé en ce que la quantité d'acide protonique fort varie de 0 à 100 moles par mole de métal catalyseur compris dans l'acide adipique et de préférence varie de 0 à 50 moles par mole de métal catalyseur.

## Claims

1. Process for crystallizing or recrystallizing adipic acid which makes it possible to reduce the content of trace metals, characterized in that the said crystallization or recrystallization is carried out in at least one carboxylic acid having a melting point of less than 20°C and in that the adipic acid which is subjected to the recrystallization has a minimum purity of 95%.

2. Process according to claim 1, characterized in that the carboxylic acid employed is selected from aliphatic carboxylic acids which are saturated or which contain an ethylenic unsaturation and, preferably, from linear or branched monocarboxylic acids having 2 to 6 carbon atoms.

3. Process according to either of claims 1 and 2, characterized in that the carboxylic acid employed is selected from acetic acid, propionic acid, butanoic acids, pentanoic acids, hexanoic acids and pentenoic acids.

4. Process according to one of claims 1 to 3, characterized in that the crystallization or recrystallization is carried out in the presence of carbon monoxide.

5. Process according to claim 4, characterized in that the carbon monoxide makes up at least part of the atmosphere above the solution in the crystallization or recrystallization reactor or creates within the said reactor a pressure which is greater than the atmospheric pressure.

6. Process according to either of claims 4 and 5, characterized in that it is carried out under an absolute pressure of from 0.5 bar to 50 bars of carbon monoxide.

7. Process according to one of claims 1 to 6, characterized in that the crude adipic acid subjected to recrystallization has a purity of from 95 to 99.95%.

8. Process according to one of claims 1 to 7, characterized in that recrystallization is carried out in the presence of a strong protic acid.

9. Process according to claim 8, characterized in that the strong protic acid is selected from hydroiodic acid, hydrobromic acid, hydrochloric acid, nitric acid and sulphuric acid.

10. Process according to one of claims 1 to 9, characterized in that the quantity of strong protic acid varies from 0 to 100 mol per mole of catalyst metal present in the adipic acid and, preferably, varies from 0 to 50 mol per mole of catalyst metal.

## Patentansprüche

1. Verfahren zur Kristallisation oder Umkristallisation von Adipinsäure, das die Verminderung des Gehalts an metallischen Spuren erlaubt, dadurch gekennzeichnet, daß diese Kristallisation oder Umkristallisation in zumindest einer Carbonsäure mit einem Schmelzpunkt unterhalb 20°C durchgeführt wird, und daß die der Umkristallisation unterzogene Adipinsäure eine minimale Reinheit von 95% besitzt.

2. Verfahren gemäß Anspruch 1, dadurch gekennzeichnet, daß die eingesetzte Carbonsäure unter den gesättigten oder eine ethylenische Unsättigung aufweisenden aliphatischen Carbonsäuren und vorzugsweise unter den linearen oder verzweigten monocarboxylischen Säuren mit 2 bis 6 Kohlenstoffatomen ausgewählt wird.

3. Verfahren gemäß einem der Ansprüche 1 oder 2, dadurch gekennzeichnet, daß die eingesetzte Carbonsäure unter Essigsäure, Propionsäure, den Butansäuren, den Pentansäuren, den Hexansäuren, den Pentensäuren, ausgewählt wird.

4. Verfahren gemäß einem der Ansprüche 1 bis 3, dadurch gekennzeichnet, daß die Kristallisation oder Umkristallisation in Gegenwart von Kohlenmonoxid durchgeführt wird.

5. Verfahren gemäß Anspruch 4, dadurch gekennzeichnet, daß das Kohlenmonoxid zumindest teilweise die über der Lösung in dem Reaktor für die Kristallisation oder Umkristallisation liegende Atmosphäre bildet oder in dem Reaktor einen Druck oberhalb Atmosphärendruck erzeugt.

6. Verfahren gemäß einem der Ansprüche 4 oder 5, dadurch gekennzeichnet, daß man bei einem absoluten Druck von 0,5 bis 50 bar Kohlenmonoxid arbeitet.

7. Verfahren gemäß einem der Ansprüche 1 bis 6, dadurch gekennzeichnet, daß die der Umkristallisation unterzogene rohe Adipinsäure eine Reinheit von 95 bis 99,95% besitzt.

8. Verfahren gemäß einem der Ansprüche 1 bis 7, dadurch gekennzeichnet, daß man die Umkristallisation in Gegenwart einer starken Protonensäure vornimmt.

9. Verfahren gemäß Anspruch 8, dadurch gekennzeichnet, daß die starke Protonensäure unter Jodwasserstoffsäure, Bromwasserstoffsäure, Chlorwasserstoffsäure, Salpetersäure, Schwefelsäure ausgewählt wird.

10. Verfahren gemäß einem der Ansprüche 1 bis 9, dadurch gekennzeichnet, daß die Menge an starker Protonensäure von 0 bis 100 Mol je Mol in der Adipinsäure enthaltenem Metallkatalysator, und vorzugsweise von 0 bis 50 Mol je Mol Metallkatalysator, variiert.
